(19) Europäisches Patentamt
European Patent Office
Office européen des brevets

(11) **EP 2 077 125 B1**

(12) **FASCICULE DE BREVET EUROPEEN**

(45) Date de publication et mention
de la délivrance du brevet:
**31.08.2011 Bulletin 2011/35**

(51) Int Cl.:
***A61L 27/44*** *(2006.01)*

(21) Numéro de dépôt: **08352027.0**

(22) Date de dépôt: **04.12.2008**

(54) **Biomatériau pour ostéosynthèse**

Biomaterialien für Osteosynthese

Biomaterial for osteosynthesis

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **07.01.2008 FR 0800077
08.01.2008 US 970596**

(43) Date de publication de la demande:
**08.07.2009 Bulletin 2009/28**

(73) Titulaires:
- **Teknimed
65502 Vic-en-Bigorre Cedex (FR)**
- **Université Paul Sabatier
31062 Toulouse Cedex 9 (FR)**
- **ARKEMA FRANCE
92700 Colombes (FR)**

(72) Inventeurs:
- **Sender, Cyril
31400 Toulouse (FR)**
- **Lacabanne, Colette
31500 Toulouse (FR)**
- **Bernes, Alain
31130 Fonsegrives (FR)**
- **Glotin, Michel
92210 Saint Cloud (FR)**

(74) Mandataire: **Morelle, Guy Georges Alain
Cabinet Morelle & Bardou, SC
Parc Technologique du Canal
9, Avenue de l'Europe
B.P. 72253
31522 Ramonville Saint Agne Cedex (FR)**

(56) Documents cités:
**WO-A-2006/074550 WO-A-2007/149300**

- **WANG ET AL: "Biocompatibility and osteogenesis of biomimetic nano-hydroxyapatite/polyamide composite scaffolds for bone tissue engineering" BIOMATERIALS, ELSEVIER SCIENCE PUBLISHERS BV., BARKING, GB, vol. 28, no. 22, 10 mai 2007 (2007-05-10), pages 3338-3348, XP022067461 ISSN: 0142-9612**
- **RUI SONG ET AL: "Preparation of Semi-aromatic polyamide(PA)/multi-wall carbon nanotube (MWCNT) composites and its dynamic mechanical properties" JOURNAL OF MATERIALS SCIENCE, KLUWER ACADEMIC PUBLISHERS, BO, vol. 43, no. 4, 12 décembre 2007 (2007-12-12), pages 1205-1213, XP019575163 ISSN: 1573-4803**

## Description

La présente invention concerne un biomatériau pour la fabrication de matériels d'ostéosynthèse doté de propriétés mécaniques dynamiques analogues à celles de l'os.

De nombreuses complications osseuses d'origine pathologique ou traumatologique sont des indications à l'utilisation de biomatériaux prothétiques. La chirurgie orthopédique représente un marché grandissant en raison du vieillissement de la population, de pathologies telles que les tumeurs osseuses et l'ostéoporose, et l'obésité qui touche de plus en plus de personnes à travers le monde.

Le matériel osseux est un composite hybride composé d'une phase organique, d'une phase minérale et d'eau, représentant respectivement en moyenne 22, 69 et 9 % en masse chez les mammifères adultes [Lee 1981, Banks 1993]. La phase organique est constituée à 90 % de substance fibrillaire (majoritairement du collagène) et de 10 % d'autres composés organiques minoritaires formant la substance dite fondamentale ou interfibrillaire [Fisher 1985, Toppets 2004]. A l'échelle moléculaire, le collagène, composant majoritaire de la phase organique osseuse est une protéine à laquelle sont associés différents niveaux de structuration. De façon générale, le collagène est constitué de chaînes polypeptidiques de 1052 à 1060 résidus liés par des liaisons peptidiques (CO-NH). Cette phase organique est à l'origine de la viscoélasticité du tissu calcifié. La phase minérale est composée de cristaux de phosphate de calcium de composition chimique proche de l'hydroxyapatite $Ca_{10}(PO_4)_6(OH)_2$ [Rey 1990]. Ce sont eux qui confèrent aux tissus calcifiés leur élasticité et leur rigidité.

Il existe deux principaux types de tissus osseux : le tissu cortical ou compact, et le tissu trabéculaire ou spongieux, représentant respectivement 80 et 20 % de la masse squelettique [Bronner 1999]. L'os compact, dit encore Haversien, apparaît comme une masse solide et dense, il est principalement responsable de la fonction de support mécanique. L'unité de base de l'os cortical est un ensemble de 20 à 30 lamelles concentriques formant un système appelé ostéon de 200 à 250 $\mu$m de diamètre en moyenne chez l'homme [Cowin 2001]. Ces ostéons s'orientent parallèlement dans l'axe de l'os (suivant les lignes du champ de contrainte mécanique) et s'associent par le biais d'os interstitiel lamellaire plus ancien provenant de la résorption d'anciens ostéons.

L'os est un matériau vivant et subit de multiples remaniements morphologiques lors de sa croissance, de son renouvellement constant (remodelage), de son vieillissement, et enfin au cours de désordres pathologiques (ostéoporose, ostéosarcome...) ou traumatologiques (fissures, fractures). Les différentes phases de formation et de résorption des tissus osseux impliquent des hormones et l'ensemble du matériel cellulaire. L'équilibre des processus dynamiques de remodelage osseux est gouverné par les champs de contraintes et les déformations subies par le squelette [Wolff 1892]. La perturbation ou la modification permanente de l'environnement mécanique d'une région osseuse aboutit à une redistribution du champ de contrainte physiologique. La réponse de l'organisme est de remanier la géométrie de l'os pour l'adapter à son nouvel environnement mécanique. Cette situation est rencontrée lorsqu'un dispositif d'ostéosynthèse est utilisé en chirurgie orthopédique [O'Doherty 1995].

Les propriétés mécaniques des tissus osseux ont fait l'objet d'un grand nombre de publications. Le tissu osseux a d'abord été considéré comme un matériau élastique caractérisé par son comportement en régime statique. Dans les conditions physiologiques, il est soumis à des contraintes dynamiques (fréquences physiologiques entre 0,1 et 10 Hz) : il a alors un comportement viscoélastique. La Spectrométrie Mécanique Dynamique (SMD) permet de le définir : une déformation $\gamma$ sinusoïdale (représentée par $\gamma^*$) est appliquée et provoque l'établissement d'une contrainte sinusoïdale $\sigma$ (représentée par $\sigma^*$) dans l'échantillon, avec un déphasage noté $\delta$.

Le module mécanique complexe en cisaillement G* est ainsi déterminé :

$$G^* = \sigma^*/\gamma^*$$

Il peut encore être défini comme la somme du module élastique ou conservatif G' et du module dissipatif ou de pertes G " :

$$G^* = G' + iG''$$

Le rapport de G " sur G', noté tan $\delta$, est le facteur de perte d'énergie mécanique.

Pour que les valeurs mesurées soient représentatives de l'état physiologique, il faut que les mesures soient effectuées sous liquide physiologique. Les valeurs du module mécanique de cisaillement G' référencées dans la littérature vont de 100 MPa à 10 GPa; les valeurs rapportées pour tan$\delta$ sont de l'ordre de $10^{-2}$.

Dans le but de se rapprocher le plus possible du comportement mécanique du tissu osseux, il faut définir un biomatériau, i.e. un matériau non vivant utilisé dans un dispositif médical destiné à interagir avec les systèmes biologiques [conférence de Chester de la Société Européenne des Biomatériaux, 1986]. Dans la grande famille des biomatériaux utilisés dans le domaine de la chirurgie orthopédique, les alliages métalliques, les céramiques et les matériaux à base polymère sont bien représentés. Chacun de ces trois groupes présente des avantages et des inconvénients qui leur sont propres.

Actuellement, la grande majorité des matériaux utilisés pour ce type d'applications (prothèses totale de hanche, de genou, matériels d'ostéosynthèse...) sont des matériaux métalliques à haut module, comparativement à celui de l'os cortical sur lequel ils sont fixés. Le champ de contrainte physiologique est dévié par ce dispositif qui supporte alors toutes les contraintes et réalise ainsi un écrantage des contraintes, appelé « stress shielding » dans la littérature [Brown 1981]. C'est le phénomène qui induit une balance de remodelage négative, donc une résorption : les zones de l'os ne remplissant plus leur rôle de soutien mécanique sont résorbées par l'organisme, et les risques de fracture après retrait de l'implant ou de son descellement à moyen terme sont accrus [Vaughan 1970, Uthoff 1971, Tonino 1976, Paavolainen 1978, Slätis 1978, Bradley 1980, Cook 1982, Uthoff 1983, Claes 1989, Huiskes 1989, Damien 1991, Huiskes 1995].

Il a été montré que l'utilisation de biomatériaux ayant des propriétés élastiques plus proches de l'os cortical permet d'accélérer les processus d'ostéogénèse [Robbins 2004]. La diminution de la protection mécanique de l'os par l'utilisation d'implants semi-rigides permet de solliciter davantage la matière osseuse tout en réduisant suffisamment la mobilité au niveau des sites fracturaires ou des interfaces céramique ostéoconductrice/plateaux vertébraux dans le cas d'arthrodèses vertébrales. Ceci a été montré récemment par la comparaison de la vitesse de fusion de vertèbres à l'aide de cages intersomatiques en titane et en polymère biodégradable [Pflugmacher 2004]. Avec ces dernières, la fusion des vertèbres est plus rapide : les contraintes mécaniques s'exerçant au niveau des plateaux vertébraux ne sont pas totalement déviées par les cages et sont transmises au matériau ostéoconducteur placé en leur centre. Il existe alors un contact intime et dynamique entre ce matériau et les vertèbres, accélérant l'ostéogénèse et la fusion.

Les biocéramiques comme la zircone, l'alumine, les phosphates de calcium, ou bien les prothèses métalliques à base de titane ou d'autres alliages, ont des modules élastiques largement supérieurs à celui de l'os cortical ou spongieux. Par exemple, le titane ou l'alliage de titane appelé Ti-6A14V utilisé pour la fabrication de prothèse totale de hanche ont un module d'Young de l'ordre de 100 GPa, et l'acier inoxydable AISI 316LTi a un module d'Young de 140 GPa [Long 1998]. Les biocéramiques ont également des modules élastiques élevés (plusieurs centaines de GPa) et sont fragiles [Ramakrishna 2001]. Notons que c'est la rigidité des implants qui est responsable du niveau de déviation des contraintes mécaniques [Brown 1979, Claes 1989]. Ce résultat a été à l'origine du développement d'implants métalliques d'épaisseur plus faible ou poreux pour diminuer leur rigidité. Mais alors, les propriétés de résistance en fatigue diminuent et les dispositifs implantés deviennent peu viables.

L'os compact a un facteur de pertes de l'ordre de $10^{-2}$. Cette caractéristique est physiologiquement fondamentale puisque c'est elle qui quantifie l'aptitude de l'os à absorber une partie de l'énergie mécanique générée lors de nos activités quotidiennes et nécessaire à son remodelage. Les biomatériaux rigides ont un facteur de pertes mécaniques tan δ inférieur à $10^{-3}$, i.e. $3,6.10^{-6}$ pour certains alliages d'aluminium [Garner 2000].

Bien que les biomatériaux métalliques actuels dits « bas module » se rapprochent des propriétés mécaniques de l'os, ils demeurent encore beaucoup plus structuraux. Les seuls dispositifs médicaux qui permettraient d'éviter la protection mécanique des tissus osseux sont des matériaux semi-rigides. C'est le concept de biomatériaux dits « analogues » introduit par Bonfield dans les années 80 [Bonfield 1981]. Une famille incontournable dans ce domaine est celle des matériaux polymères, bien connue pour leur aptitude d'absorption par dissipation visqueuse. Pour être mécaniquement biocompatible, les systèmes macromoléculaires doivent posséder à la fois des propriétés élastiques élevées et des propriétés d'absorption mécanique comparables à la matière osseuse.

Des dispositifs d'ostéosynthèse à base de polymères synthétiques non biorésorbables ont fait l'objet d'essais sur animal. Ces derniers ayant généralement des propriétés mécaniques intrinsèques inférieures à celles de l'os, ils ont été renforcés. Les composites obtenus admettent un comportement visqueux similaire aux tissus calcifiés et des modules élastiques généralement plus faibles que l'os. A titre d'illustration, citons les plaques en polytrifluoromonochloroethylène (PTFCE) de Tonino et al. [Tonino 1976], les plaques d'ostéosynthèse semi-rigides à base de polysulfone/graphite et epoxy/verre de Bradley et al. [Bradley 1977]. Des plaques en polymères chargés en fibres de carbone ayant des modules d'élasticité allant de 2 à 3.5 GPa ont été testées en torsion statique [Claes 1980]. L'implantation de ces plaques sur animal a posé des problèmes en résistance à la rupture et n'a pas été concluante.

Les premiers cas d'implantation de plaques d'ostéosynthèse semi-rigides sur des êtres humains ont été reportés par Tayton et al. [Tayton 1982]. Des plaques multiaxiales en résine époxy renforcée de fibres de carbone ont été implantées sur des patients souffrant de fractures : L'os se répare rapidement et atteint une rigidité normale en seulement 25 semaines. Pour la fixation d'un tibia fracturé, Tayton et Bradley iront jusqu'à proposer une rigidité optimale de la plaque d'ostéosynthèse de 2,0 N.m par degrés [Tayton 1983].

De nombreux autres composites ont été élaborés et étudiés pour des applications en orthopédie. Parmi ceux-ci, on trouve des matrices polymères chargées avec des particules d'HAp comme dans le cas du Polyéthylène Haute Densité [Bonfield 1981, Tanner 1992, Wang 1994, Deb 1996, Wang 1998, Roeder 2003], de polylactides [Verheyen 1992, Kikuchi 1997, Zhang 1999, Shikinami 1999, Ignjatovic 1999, Durucan 2000], du PMMA [Ravaglioli 1992, Kazuhiko 1992, Harper 2000], du Poly(acide acrylique) [Liou 2003]... D'autres, ont été renforcés à l'aide de fibres longues ou courtes de carbone. Bien que cet élément ait une excellente biocompatibilité (totalement inerte), le relargage *in vivo* de particules d'usure dans les tissues environnants a donné de mauvais résultats [Claes 1983]. Les extrémités de fibres de carbone en surface des implants sont extrêmement abrasive et irritantes [Evans 1998]. Wan et al. ont également montré que, malgré l'inertie chimique de fibres de carbure de silicium, leur niveau de cytotoxicité en contact direct avec des cellules est élevé [Wan 1993].

L'utilisation de matériaux structuraux capables d'absorber une partie de l'énergie mécanique n'est donc plus à démontrer. Le concept de semi-rigidité a longtemps suscité l'intérêt dans le domaine de l'orthopédie. Mais aujourd'hui encore, les métaux et en particulier les implants à base de titane sont largement utilisés faute de matériaux semi-rigides ayant des modules élastiques entrant dans la gamme des tissus osseux.

Afin de résoudre le problème technique de la résistance à la contrainte des polymères utilisés comme biomatériaux, l'introduction de cycles aromatiques dans la structure de chaîne du polymère a été envisagée pour augmenter ses propriétés physiques. De tels matériaux actuellement en développement pour des applications automobiles, n'ont jamais été envisagés dans le domaine médical compte tenu de l'écart des problématiques rencontrées dans ces deux secteurs.

Des polymères industriels techniques ont été développés à partir de polyamides aromatiques , dès les années 60. L'un des plus connus est le Kevlar ou Poly-para-phénylène téréphtalamide mis au point par la société Du Pont de Nemours en 1965. Ce matériau allie de très hautes propriétés mécaniques associées à des capacités importantes d'absorption des chocs et une excellente résistance à la fatigue et à de nombreux solvants. Ses applications sont variées : matériels de protection (casques, gilets), aéronautique et aérospatial, matériels sportifs... Ses propriétés mécaniques étant très élevées et sa mise en oeuvre non aisée, certains industriels ont développé des polyamides ayant une composition intermédiaire entre celle des polyamides aromatiques et des polyamides aliphatiques tels que le Polyamide 6 (PA6) ou le Polyamide 11 (PA11). Ce sont les polyamides dits semi-aromatiques PASAs. Le contrôle de la teneur relative en cycle aromatique dans la structure de chaîne permet d'ajuster les propriétés physiques de ces polymères. Alliant les propriétés remarquables d'amortissement des polyamides aux propriétés mécaniques et thermiques élevées des polymères aromatiques, la famille des PASAs permet de répondre à un grand nombre d'applications. Une recherche industrielle active a abouti à la commercialisation de nombreux PASAs comme le Cristamid® d'Arkema à base de PA12, le IXEF® de Solvay, le PA6/6T ou Ultramid T® de BASF, le Zytel® de Du Pont, le PA9T ou Genestar® de Kuraray, le Grilamid® d'E.M.S, le Trogamid® de Evonik...

Dans le domaine biomédical, seuls des polyamides aliphatiques ont été utilisés dans des applications diverses telles que des fils de suture, membranes de dialyse [Yamashita 1996], peau artificielle [Bugmann 1998, Mei 2003], milieu de culture cellulaire [Catapano 1996], cathéters, seringues... La biocompatibilité des matériaux polyamides s'explique par la similarité de leur composition chimique avec les protéines naturelles comme les collagènes [Risbud 2001, Jie 2001]. En effet, les groupements amides contenus dans les polyamides sont identiques aux liaisons peptidiques dans les protéines. L'expression « polyamide naturel » a même été utilisée par Das et al. pour qualifier la gélatine, produit issu de la dénaturation du collagène [Das 2003].

Le niveau de cytotoxicité du polyamide 6 utilisé pour la fabrication de supports de culture cellulaire en ingénierie tissulaire est faible [Das 2003]. L'implantation de polyamide 66 chargé en hydroxyapatite a donné des résultats spécifiquement intéressants en terme de biocompatibilité [Xiang 2002]. Cependant, son hydrophilie provoque une chute des propriétés mécaniques à l'état hydraté.

Afin de répondre aux inconvénients de l'art antérieur, la présente invention propose un biomatériau pour la fabrication de matériels d'ostéosynthèse aux propriétés mécaniques dynamiques analogues au tissu calcifié, caractérisé en ce qu'il comprend une matrice polyamide semi-aromatique hydrophobe et au moins un renfort.

Le terme renfort désigne tout composé capable d'optimiser les propriétés mécaniques de la matrice. De morphologie variable, le renfort utilisé dans la présente invention peut présenter un aspect particulier, c'est à dire avec des dimensions du même ordre de grandeur, i.e.entre 10 nm et 100 $\mu$m.

La taille des particules renforçantes est un facteur crucial pour l'obtention de l'effet renforçant : plus la surface développée entre la matrice et les renforts est élevée, meilleur sera le transfert de contrainte mécanique. Ainsi, l'utilisation de particules de dimensions nanométriques permet d'augmenter considérablement la surface de contact entre les deux phases. Une forme particulièrement avantageuse de renfort particulaire consiste en des aiguilles ou des lamelles pouvant être associées.

Dans le cas de renfort non particulaire, un aspect fibreux est également couvert par la présente invention. Le renfort est alors défini par son facteur de forme Longueur (L) rapporté au diamètre (d) avec des valeurs supérieures à 10. L'utilisation de renforts ayant un facteur de forme élevé optimise les propriétés mécaniques des composites.

De manière préférée, le renfort consistera en des composés inorganiques choisis parmi des verres, des silicates, des phosphates de calcium et un mélange de ceux-ci.

Dans une logique biomimétique, le matériau choisi pour renforcer la matrice polyamide est l'hydroxyapatite ou HAp. Le caractère hydrophile (polaire) des matériaux apatitiques permet la formation de liaisons physiques avec les groupements polaires de la matrice polyamide, indispensables pour le transfert des charges mécaniques de la matrice aux renforts

Le renfort peut également être un composé organique choisi de préférence parmi des polyamides ou du carbone et un mélange de ceux-ci.

La matrice polyamide semi-aromatique selon l'invention comprend au moins un homopolyamides de formule **Y.Ar** avec :

- **Y** désigne un motif provenant d'au moins une diamine aliphatique et/ou cycloaliphatique, saturée, linéaire ou ramifiée ayant de préférence de 4 à 20 atomes de carbone,

- **Ar** désigne un motif provenant d'au moins un diacide carboxylique aromatique éventuellement substitué ayant de préférence de 8 à 22 atomes de carbone, ledit diacide carboxylique aromatique étant avantageusement un acide phtalique, l'acide phtalique étant de préférence choisi parmi l'acide téréphtalique, l'acide isophtalique, l'acide orthophtalique et leurs mélanges.

Elle peut également comprendre au moins un copolyamide de formule **X/Y.Ar** avec :

- **Y** désigne un motif provenant d'au moins une diamine aliphatique et/ou cycloaliphatique, saturée, linéaire ou ramifiée ayant de préférence de 4 à 20 atomes de carbone,
- **Ar** désigne un motif provenant d'au moins un diacide carboxylique aromatique éventuellement substitué ayant de préférence de 8 à 22 atomes de carbone, ledit diacide carboxylique aromatique étant avantageusement un acide phtalique, l'acide phtalique étant de préférence choisi parmi l'acide téréphtalique, l'acide isophtalique, l'acide orthophtalique et leurs mélanges.
- **X** désigne :

  - soit un motif provenant d'au moins un lactame et/ou d'au moins un alpha-oméga aminoacide carboxylique, le lactame et/ou l'alpha-oméga aminoacide carboxylique comprenant de préférence de 6 à 18 atomes de carbone,
  - soit un motif **U.V** provenant de la condensation d'au moins une diamine U avec au moins un diacide carboxylique V,

    • la diamine **U** linéaire ou ramifiée étant choisie parmi une diamine aliphatique, une diamine cycloaliphatique, une diamine aromatique et leurs mélanges et comprenant de préférence de 4 à 20 atomes de carbone,
    • le diacide carboxylique **V** linéaire ou ramifié étant choisi parmi un diacide aliphatique, un diacide cycloaliphatique, un diacide aromatique et leurs mélanges, et comprenant de préférence de 6 à 20 atomes de carbone.

De préférence, le nombre d'atomes de carbones de l'un au moins des motifs X et Y est compris entre 6 et 12.

On choisit de préférence Y et U parmi le groupe suivant : 1,6-hexaméthylènediamine, 1,9-nonanediamine, 2-méthyl-1,8-octanediamine, 1,10-décanediamine, 1,12-dodécanediamine, et leurs mélanges.

X comprend de préférence le lactame 12, l'amino-11-undécanoïque, l'acide amino-12-dodécanoïque et leurs mélanges.

V est choisi de préférence parmi le groupe suivant : acide adipique, acide subérique, acide azélaïque, acide sébacique, diacide 1,12 dodécanedioïque, acide brassylique, diacide 1,14-tétradécanedioïque, acide téréphtalique, acide isophtalique, acide naphtalènedicarboxylique, et leurs mélanges.

Les proportions molaires de X par rapport à Y (ou Ar) sont pour Y = 1, $0 \leq X \leq 0,7$, préférentiellement $0 \leq X \leq 0,5$.

Les diamines Y et U peuvent être identiques ou non.

Dans les formules **Y.Ar** et **X/Y.Ar,** les expressions « au moins une diamine » et « au moins un diacide » signifient respectivement et indépendamment l'une de l'autre « un, deux ou trois diamine(s) » et « un, deux ou trois diacide(s) ».

Le biomatériau selon la présente invention comprend jusqu'à 70 % de renfort par rapport à la masse totale de biomatériau. Bien que facultatif, il peut comprendre un agent ou un mélange d'agents surfactants, une molécule ou un mélange de molécules amphiphiles ou tout autre agent ou mélange d'agents compatibilisants. A titre d'exemple citons les polyéthylènes « glycol », des acides gras comme l'acide palmitique...

Pour optimiser les propriétés mécaniques du biomatériau, celui-ci doit comporter un pourcentage d'eau ajoutée inférieur à 5 % en masse totale. Si nécessaire, une étape complémentaire de séchage du biomatériau est effectuée pour atteindre ce pourcentage d'eau.

Le biomatériau ainsi défini se caractérise par des propriétés mécaniques dynamiques analogues au tissu calcifié. Ces propriétés correspondent à un niveau de viscoélasticité significatif aux températures (37°C) et fréquences physiologiques (0,1 à 10 Hz) défini par un module conservatif et un facteur de perte d'énergie mécanique de l'ordre de ceux du tissu calcifié.

Les valeurs du module conservatif représenté par G' correspondant au biomatériau selon l'invention sont ainsi comprises entre 100 MPa et 10 GPa, en mode cisaillement.

Les valeurs du facteur de perte d'énergie mécanique représenté par tan δ sont supérieures à $10^{-3}$ en mode cisaillement.

Le biomatériau selon la présente invention est particulièrement destiné à la fabrication de dispositifs d'ostéosynthèse ou de prothèses dentaires. De manière plus large, il peut être utilisé dans toute application médicale nécessitant des composés dotés de propriétés mécaniques proches du tissu osseux.

Les propriétés du biomatériau selon l'invention sont mises en évidence à travers les figures suivantes :

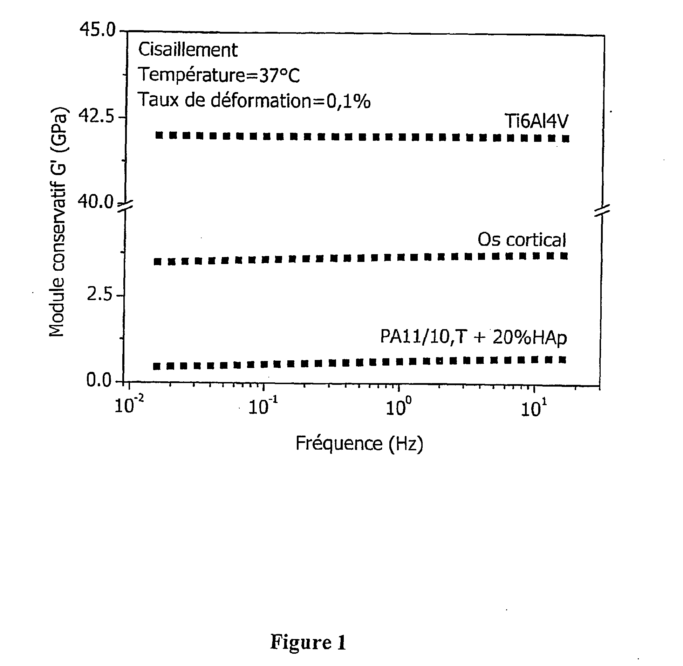

- Figure 1 : illustre le module conservatif G' en fonction de la fréquence d'un biomatériau selon l'invention comprenant une matrice polyamide semi-aromatique à base de PA11/10,T et un taux de renfort de 20 % d'HAp. Ces valeurs sont comparées à celles d'un os cortical ainsi qu'un matériau en alliage Ti6Al4V.

  On remarque que le module conservatif G' du biomatériau selon l'invention se situe dans la zone de valeur de celui de l'os cortical alors que celui du matériau en alliage Ti6Al4V est dix fois plus élevé.

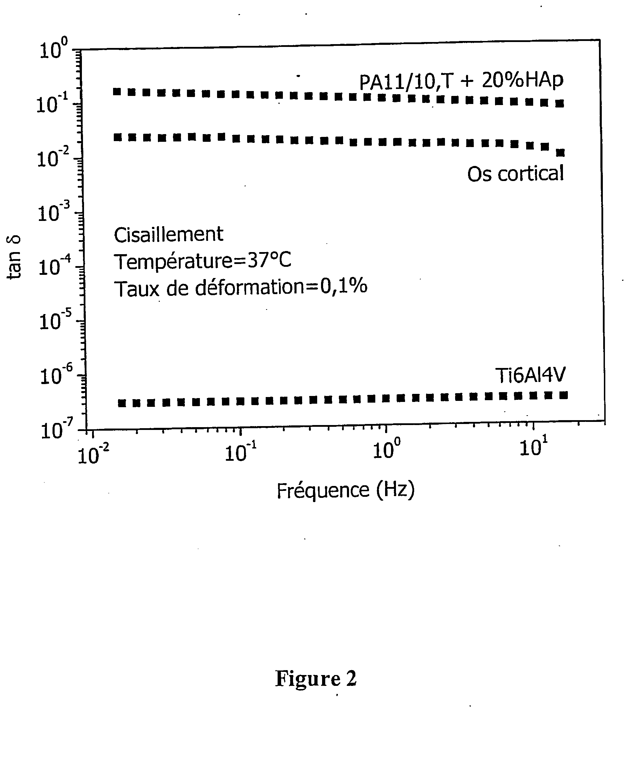

- Figure 2 : illustre le facteur de perte d'énergie mécanique tan δ en fonction de la fréquence d'un biomatériau selon l'invention comprenant une matrice polyamide semi-aromatique à base de PA11/10,T et un taux de renfort de 20 % d'HAp. Ces valeurs sont comparées à celles d'un os cortical ainsi qu'un matériau en alliage Ti6Al4V.

  Le facteur de perte d'énergie mécanique du biomatériau selon l'invention se situe dans la zone de valeur de celui de l'os cortical alors que celui du matériau en alliage Ti6Al4V est très éloigné.

Les exemples qui suivent visent à illustrer la présente invention sans en limiter la portée.

EXEMPLE 1

**Mise en oeuvre d'un biomatériau selon l'invention en dispersion par voie solvant**

■ substitution de solvant:
Les polyamides ne pouvant être dissous dans le DMAc lorsque le milieu contient de l'eau, l'eau de la suspension nHAp/eau est substituée par du DMAc.
■ désagglomération des particules de nHAp:
La quantité souhaitée de suspension nHAp/DMAc est agitée et sonifiée à l'aide d'une sonde à ultrasons 500W/20kHz de chez Sonics avec une amplitude de vibration fixée à 95% de l'amplitude maximale de l'appareil. La naissance puis l'explosion de microbulles au sein de la suspension engendre la libération d'une énergie considérable (phénomène de cavitation) assurant une agitation intense du milieu permettant de briser les agglomérats.
■ dissolution du PASA:
La quantité souhaitée de PASA est versée dans la suspension de nHAp et dissoute.
■ précipitation, filtration et lavage du nanocomposite:
De l'eau distillée, un non-solvant du polyamide, est versée pour précipiter le nanocomposite. Une suspension de particules millimétriques de nanocomposite, dans un milieu liquide, mélange d'eau et de DMAc, est obtenue. L'affinité entre l'eau et le DMAc est supérieure à celle entre le nanocomposite et le DMAc de sorte que l'eau en large excès dans le milieu se substitue au DMAc dans le nanocomposite [Kasowski 1994]. Le tout est ensuite filtré sur filtre Büchner et abondamment lavé à l'eau distillée. Le produit obtenu est une pâte blanche fortement saturée en eau. Elle est séchée dans une étuve.
■ broyage :
Le nanocomposite séché se présente sous forme de gros agrégats centimétriques. L'injection de cette matière nécessite une étape de broyage préliminaire. Les nanocomposites sont trempés à l'azote, et un broyeur de type ZM100 de chez Retsch est utilisé pour obtenir une poudre fine.

EXEMPLE 2

**Etude de la cytotoxicité d'un polyamide semi-aromatique utilisé comme biomatériau selon l'invention : le PA11/10,T**

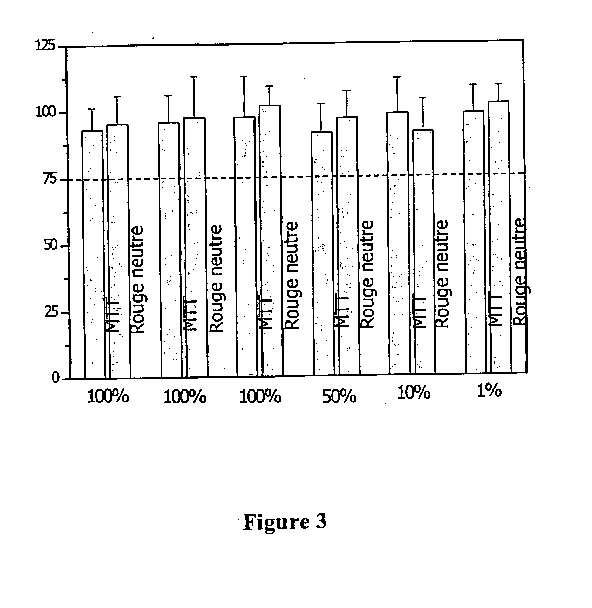

Le PA11/10,T fourni par la société Arkema, se présente sous forme de granulés légèrement opaques. C'est un polymère statistique synthétisé par polycondensation de trois monomères, l'acide 11-aminoundecanoïque, le decamethylènediamine et l'acide téréphtalique. Le PA11/10,T est un polymère semi-cristallin ayant une température de transition vitreuse de l'ordre de 80°C et une fusion sur une plage de températures de 200/270°C, en fonction de la proportion molaire d'acide 11-aminoundecanoïque par rapport à celle de decamethylèneciamine (ou d'acide téréphtalique). Le PA11/10,T absorbe environ 1,2 et 2% en masse d'eau lorsqu'il est respectivement maintenu aux conditions ambiantes ou hydraté à saturation dans de l'eau distillée.
La cytotoxicité du PA11/10,T a été déterminée sur des cultures de cellules ostéoprogénitrices humaines issues de stroma médullaire au Laboratoire de Biophysique de l'Université Victor Segalen à Bordeaux. Une étude de précontamination microbienne avant la stérilisation ainsi que la détermination de la teneur résiduelle en oxyde d'éthylène après stérilisation ont montré que le PA11/10,T a été correctement conditionné et stérilisé. Le test du MTT caractérisant l'activité métabolique des cellules et le test du Rouge neutre témoin de la viabilité cellulaire ont été réalisés. Des extraits du PA11/10,T à 100% puis dilués à 50, 10 et 1% ont été testés. Un matériau est considéré comme cytotoxique si les valeurs obtenues sont en dessous de 75% par rapport aux cultures témoins. Les résultats des tests représentés sur la Figure 3 montrent que le PA11/10,T n'est pas cytotoxique.

EXEMPLE 3

**Dispositif expérimental utilisé pour la mesure du module mécanique dynamique de cisaillement G*: Spectrométrie Mécanique Dynamique (SMD)**

Les essais sont réalisés à l'aide d'un rhéomètre ARES de chez Thermal Analysis Instruments. Le mode de sollicitation choisi est la torsion rectangulaire à taux de déformation imposé. Un moteur solidaire de l'extrémité inférieure de l'échantillon impose un mouvement de torsion alors que le couple induit sur le mors supérieur par l'intermédiaire de l'échantillon est enregistré par une cellule de mesure. Ce couple de torsion est ensuite converti en contrainte.

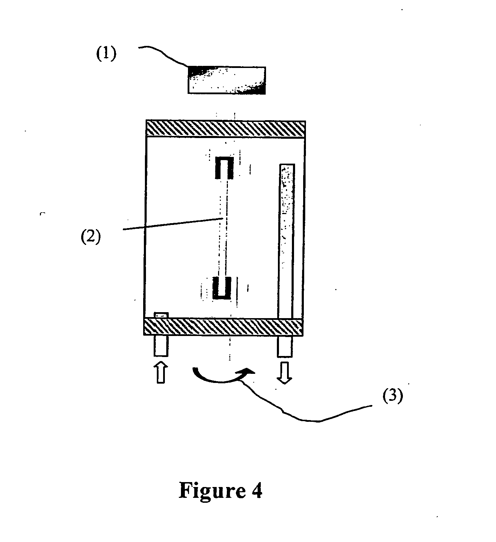

Les échantillons peuvent être sollicités sous air (dans un four) ou immergés dans une solution aqueuse à l'aide d'une cellule dans laquelle le fluide circule (Figure 4 illustrant un dispositif de torsion rectangulaire dans la cellule liquide thermostatée, avec cellule de mesure du couple (1), échantillon (2), déformation imposée (3)). Sous air, la température peut varier entre -140 et 300°C. Les basses températures sont accessibles par l'utilisation d'un réservoir d'azote liquide. En solution aqueuse, le domaine de température est restreint à 10/80°C. C'est un cryothermostat Julabo F25 qui contrôle alors la température du fluide circulant.

Les échantillons ont une forme parallélépipédique de largeur b, d'épaisseur a et de longueur L telles que a<<b et b<L. Un facteur de forme K est défini:

$$K = \frac{3L}{ab^3} \times \frac{1}{1 - 0.63\frac{b}{a}}$$

Ce facteur permet de relier la contrainte complexe $\sigma^*(\omega)$ et le module mécanique dynamique $G^*(\omega)$:

$$G^*(\omega) = K\sigma^*(\omega) = K\frac{T_0}{\theta^*(\omega)}e^{i\delta(\omega)}$$

avec $T_0$ le couple de torsion mesuré par le mors supérieur et $\theta^*(\omega)$ l'angle de déformation de l'extrémité inférieure de l'échantillon.

La force axiale a été minimisée avant le lancement des tests pour ne pas modifier les valeurs des pertes mécaniques. En effet, une contrainte axiale en traction sur un échantillon d'os cortical soumis à une sollicitation en torsion ne modifie pas significativement son module mais peut augmenter les pertes mécaniques [Lakes 1979].

**Revendications**

1.  Biomatériau pour la fabrication de matériels d'ostéosynthèse aux propriétés mécaniques dynamiques analogues au tissu calcifié, **caractérisé en ce qu'**il comprend :

    - une matrice polyamide semi-aromatique,
    - au moins un renfort.

2.  Biomatériau selon la revendication 1, **caractérisé en ce que** ledit renfort possède un aspect particulaire avec une dimension comprise entre 10 nm et 100 $\mu$m.

3.  Biomatériau selon la revendication 2, **caractérisé en ce que** ledit renfort particulaire se présente sous la forme d'aiguilles et/ou de lamelles.

4.  Biomatériau selon la revendication 1, **caractérisé en ce que** ledit renfort possède un aspect fibreux avec un facteur de forme L/d supérieur à 10.

5.  Biomatériau selon l'une quelconque des revendications précédentes, **caractérisé en ce que** ledit renfort est un composé inorganique choisi parmi des verres, des silicates, des phosphates de calcium et un mélange de ceux-ci.

6.  Biomatériau selon la revendication 5, **caractérisé en ce que** ledit renfort est une apatite.

7.  Biomatériau selon l'une quelconque des revendications 1 à 4, **caractérisé en ce que** ledit renfort est un composé organique choisi parmi des polyamides, du carbone et leur mélange.

8.  Biomatériau selon l'une quelconque des revendications précédentes, **caractérisé en ce que** la matrice polyamide semi-aromatique comprend au moins un homopolyamide de formule **Y.Ar** avec :

    - **Y** désigne un motif provenant d'au moins une diamine aliphatique et/ou cycloaliphatique, saturée, linéaire ou ramifiée ayant de préférence de 4 à 20 atomes de carbone,
    - **Ar** désigne un motif provenant d'au moins un diacide carboxylique aromatique éventuellement substitué ayant de préférence de 8 à 22 atomes de carbone.

9.  Biomatériau selon l'une quelconque des revendications 1 à 8, **caractérisé en ce que** la matrice polyamide semi-aromatique comprend au moins un copolyamide de formule **X/Y.Ar** avec :

    - **Y** désigne un motif provenant d'au moins une diamine aliphatique et/ou cycloaliphatique, saturée, linéaire ou ramifiée ayant de préférence de 4 à 20 atomes de carbone,

- **Ar** désigne un motif provenant d'au moins un diacide carboxylique aromatique éventuellement substitué ayant de préférence de 8 à 22 atomes de carbone, et

- **X désigne :**

    - soit un motif provenant d'au moins un lactame et/ou d'au moins un alpha-oméga aminoacide carboxylique, le lactame et/ou l'alpha-oméga aminoacide carboxylique comprenant de préférence de 6 à 18 atomes de carbone,

    - soit un motif **U.V** provenant de la condensation d'au moins une diamine **U** avec au moins un diacide carboxylique **V,**

        la diamine **U** linéaire ou ramifiée étant choisie parmi une diamine aliphatique, une diamine cycloaliphatique, une diamine aromatique et leurs mélanges et comprenant de préférence de 4 à 20 atomes de carbone, le diacide carboxylique **V** linéaire ou ramifié étant choisi parmi un

        diacide aliphatique, un diacide cycloaliphatique, un diacide aromatique et leurs mélanges, et comprenant de préférence de 6 à 20 atomes de carbone.

10. Biomatériau selon la revendication 8 ou 9, **caractérisé en ce que** le diacide carboxylique aromatique Ar est un acide phtalique, de préférence choisi parmi l'acide téréphtalique, l'acide isophtalique, l'acide orthophtalique et leurs mélanges.

11. Biomatériau selon l'une quelconque des revendications 9 à 10, **caractérisé en ce que** le nombre d'atomes de carbone de l'un au moins des motifs X et Y comprend de 6 à 12 atomes de carbone.

12. Biomatériau selon l'une quelconque des revendications 9 à 11, **caractérisé en ce que** l'un au moins du motif Y et de la diamine U est choisi parmi le groupe suivant : 1,6-hexaméthylènediamine, 1,9-nonanédiamine, 2-méthyl-1,8-octanediamine, 1,10-décanediamine, 1,12-dodécanediamine et leurs mélanges.

13. Biomatériau selon l'une quelconque des revendications 9 à 12, **caractérisé en ce que** X est choisi parmi le lactame 12, l'amino-11-undécanoïque, l'acide amino-12-dodécanoïque et leurs mélanges.

14. Biomatériau selon l'une quelconque des revendications 9 à 13, **caractérisé en ce que** V est choisi parmi le groupe suivant : acide adipique, acide subérique, acide azélaïque, acide sébacique, diacide 1,12 dodécanedioïque, acide brassylique, diacide 1,14-tétradécanedioïque, acide téréphtalique, acide isophtalique, acide naphtalènedicarboxylique et leurs mélanges.

15. Biomatériau selon l'une quelconque des revendications 9 à 14, **caractérisé en ce que** les proportions molaires de X par rapport à Y (ou Ar) sont :

    pour Y = 1,
    $0 \leq X \leq 0,7$.

16. Biomatériau selon la revendication 15, **caractérisé en ce que** les proportions molaires de X par rapport à Y (ou Ar) sont de préférence :

    pour Y =1,
    $0 \leq X \leq 0,5$.

17. Biomatériau selon l'une quelconque des revendications 9 à 16, **caractérisé en ce que** les diamines Y et U sont identiques.

18. Biomatériau selon l'une quelconque des revendications précédentes, **caractérisé en ce qu'**il comprend jusqu'à 70 % en masse, par rapport à la masse totale de biomatériau, de renfort.

19. Biomatériau selon l'une quelconque des revendications précédentes, **caractérisé en ce qu'**il comprend en outre un agent ou un mélange d'agents surfactants, une molécule ou un mélange de molécules amphiphiles ou tout autre agent ou mélange d'agents compatibilisants.

20. Biomatériau selon l'une quelconque des revendications précédentes, **caractérisé en ce qu'**il comprend un pourcentage d'eau ajoutée inférieur à 5 % en masse, par rapport à la masse totale de biomatériau.

21. Biomatériau selon l'une quelconque des revendications précédentes, **caractérisé en ce que** la matrice polyamide semi-aromatique et le renfort sont choisis de sorte que les propriétés mécaniques dynamiques du biomatériau répondent à un niveau de viscoélasticité significatif aux températures et fréquences physiologiques de l'ordre de celui du tissu calcifié, défini par un module conservatif G' compris entre 100 MPa et 10 GPa (bornes comprises), en mode de cisaillement, et un facteur de perte d'énergie mécanique représenté par tan $\delta$ supérieur à $10^{-3}$ en mode de cisaillement.

22. Utilisation du biomatériau selon l'une quelconque des revendications précédentes pour la fabrication de dispositifs d'ostéosynthèse ou de prothèses dentaires.

## Claims

1. Biomaterial for the manufacture of osteosynthesis articles having dynamic mechanical properties analogous to calcified tissue, **characterised in that** it includes:

   - a semi-aromatic polyamide matrix, and
   - at least one reinforcing means.

2. Biomaterial according to claim 1, **characterised in that** said reinforcing means possesses a particular appearance with a dimension of between 10 nm and 100 μm.

3. Biomaterial according to claim 2, **characterised in that** said particular reinforcing means is in the form of needles and/or strips.

4. Biomaterial according to claim 1, **characterised in that** said reinforcing means possesses a fibrous appearance with an L/d shape factor greater than 10.

5. Biomaterial according to any of the preceding claims, **characterised in that** said reinforcing means is an inorganic compound selected from glasses, silicates, calcium phosphates and a mixture thereof.

6. Biomaterial according to claim 5, **characterised in that** said reinforcing means is an apatite.

7. Biomaterial according to any of claims 1 to 4, **characterised in that** said reinforcing means is an organic compound selected from polyamides, carbon and their mixture.

8. Biomaterial according to any of the preceding claims, **characterised in that** the semi-aromatic polyamide matrix includes at least one homopolyamide of the formula **Y.Ar** with:

   - **Y** representing an element comprising at least one saturated, linear or branched aliphatic and/or cycloaliphatic diamine having preferably between 4 and 20 carbon atoms, and
   - **Ar** representing an element comprising at least one possibly substituted aromatic carboxylic diacid having preferably between 8 and 22 carbon atoms.

9. Biomaterial according to any of claims 1 to 8, **characterised in that** the semi-aromatic polyamide matrix includes at least one copolyamide of the formula **X/Y.Ar** with:

   - **Y** representing an element comprising at least one saturated, linear or branched aliphatic and/or cycloaliphatic diamine having preferably between 4 and 20 carbon atoms,
   - **Ar** representing an element comprising at least one possibly substituted aromatic carboxylic diacid having preferably between 8 and 22 carbon atoms, and
   - **X** representing:

     - either an element comprising at least one lactam and/or at least one carboxylic alpha-omega aminoacid, the lactam and/or the carboxylic alpha-omega aminoacid having preferably between 6 and 18 carbon atoms,
     - or an element **U.V** arising from the condensation of at least one diamine U with at least one carboxylic diacid **V,**

       . the linear or branched diamine **U** being selected from an aliphatic diamine, a cycloaliphatic diamine, an aromatic diamine and their mixtures and having preferably between 4 and 20 carbon atoms, and
       . the linear or branched carboxylic diacid **V** being selected from an aliphatic diacid, a cycloaliphatic diacid, an aromatic diacid and their mixtures, and having preferably between 6 and 20 carbon atoms.

10. Biomaterial according to claim 8 or 9, **characterised in that** the aromatic carboxylic diacid Ar is a phthalic acid, preferably selected from terephthalic acid, isophthalic acid, orthophthalic acid and their mixtures.

11. Biomaterial according to any of claims 9 to 10, **characterised in that** the number of carbon atoms of one at least of the elements X and Y is between 6 and 12 carbon atoms.

12. Biomaterial according to any of claims 9 to 11, **characterised in that** one at least of the element Y and of the diamine U is selected from the following group: 1,6-hexamethylene diamine, 1,9-nonane diamine, 2-methyl-1,8-octane diamine, 1,10-decane diamine, 1,12-dodecane diamine and their mixtures.

13. Biomaterial according to any of claims 9 to 12, **characterised in that** X is selected from lactam 12 amino-11-undecanoic acid, amino-12-dodecanoic acid and their mixtures.

14. Biomaterial according to any of claims 9 to 13, **characterised in that** V is selected from the following group: adipic acid, suberic acid, azelaic acid, sebacic acid, 1,12-dodecanedioic diacid, brassylic acid, 1,14-tetradecanedioic diacid, terephthalic acid, isophthalic acid, naphthalene dicarboxylic acid and their mixtures.

15. Biomaterial according to any of claims 9 to 14, **characterised in that** the molar proportions of X relative to Y (or Ar) are:

for Y=1,
$0 \leq X \leq 0.7$.

16. Biomaterial according to claim 15, **characterised in that** the molar proportions of X relative to Y (or Ar) are preferably:

for Y = 1,
$0 \leq X \leq 0.5$.

17. Biomaterial according to any of claims 9 to 16, **characterised in that** the diamines Y and U are identical.

18. Biomaterial according to any of the preceding claims, **characterised in that** it includes up to 70 % by weight of reinforcing means relative to the total weight of the biomaterial.

19. Biomaterial according to any of the preceding claims, **characterised in that** it includes in addition a surfactant agent or a mixture of surfactant agents, an amphiphilic molecule or a mixture of amphiphilic molecules or any other compatibilising agent or mixture of compatibilising agents.

20. Biomaterial according to any of the preceding claims, **characterised in that** it includes a percentage of added water of less than 5 % by weight, relative to the total weight of biomaterial.

21. Biomaterial according to any of the preceding claims, **characterised in that** the semi-aromatic polyamide matrix and the reinforcing means are selected so that the dynamic mechanical properties of the biomaterial comply with a significant level of viscoelasticity at physiological temperatures and frequencies of the same order as that of the calcified tissue, said level being defined by a preservative modulus G' ranging between 100 MPa and 10 GPa (limits included) in the shearing mode, and a mechanical energy loss factor, represented by tan $\delta$, greater than $10^{-3}$ in the shearing mode.

22. Use of the biomaterial according to any of the preceding claims for the manufacture of osteosynthesis devices or dental prostheses.

**Patentansprüche**

1. Biomaterial für die Herstellung von Osteosynthesematerialien mit dynamischen mechanischen Eigenschaften, die jenen eines verkalkten Gewebes analog sind, **dadurch gekennzeichnet, dass** es

- eine halbaromatische Polyamidmatrix und
- mindestens eine Verstärkung

umfasst.

2. Biomaterial nach Anspruch 1, **dadurch gekennzeichnet, dass** die Verstärkung einen partikulären Aspekt mit einer Abmessung zwischen 10 nm und 100 $\mu$m aufweist.

3. Biomaterial nach Anspruch 2, **dadurch gekennzeichnet, dass** die partikuläre Verstärkung die Form von Nadeln und/oder Lamellen hat.

4. Biomaterial nach Anspruch 1, **dadurch gekennzeichnet, dass** die Verstärkung einen fasrigen Aspekt mit einem Formfaktor L/d höher als 10 aufweist.

5. Biomaterial nach einem der vorhergehenden Ansprüche, **dadurch gekennzeichnet, dass** die Verstärkung eine anorganische Verbindung ist, die unter Gläsern, Silikaten, Calciumphosphaten und einer Mischung davon ausgewählt ist.

6. Biomaterial nach Anspruch 5, **dadurch gekennzeichnet, dass** die Verstärkung ein Apatit ist.

7. Biomaterial nach einem der Ansprüche 1 bis 4, **dadurch gekennzeichnet, dass** die Verstärkung eine organische Verbindung ist, die unter Polyamiden, Kohlenstoff und einer Mischung davon ausgewählt ist.

8. Biomaterial nach einem der vorhergehenden Ansprüche, **gekennzeichnet dadurch, dass** die halbaromatische Polyamidmatrix mindestens ein Homopolyamid der Formel Y.Ar umfasst, wobei:

- Y ein Muster bezeichnet, welches aus mindestens einem gesättigten, geradkettigen oder verzweigten aliphatischen und/oder cycloaliphatischen Diamin mit vorzugsweise 4 bis 20 Kohlenstoffatomen abgeleitet ist,
- Ar ein Muster bezeichnet, welches aus mindestens einer gegebenenfalls substituierten aromatischen zweiwertigen Carbonsäure mit vorzugsweise 8 bis 22 Kohlenstoffatomen abgeleitet ist.

9. Biomaterial nach einem der Ansprüche 1 bis 8, **da-**

**durch gekennzeichnet, dass** die halbaromatische Polyamidmatrix mindestens ein Copolyamid der Formel X/Y.Ar umfasst, wobei:

- Y ein Muster bezeichnet, welches aus mindestens einem gesättigten, geradkettigen oder verzweigten aliphatischen und/oder cycloaliphatischen Diamin mit vorzugsweise 4 bis 20 Kohlenstoffatomen abgeleitet ist,
- Ar ein Muster bezeichnet, welches aus mindestens einer gegebenenfalls substituierten aromatischen zweiwertigen Carbonsäure mit vorzugsweise 8 bis 22 Kohlenstoffatomen abgeleitet ist, und
- X bezeichnet:

- entweder ein Muster, welches aus mindestens einem Lactam und/oder mindestens einer Alpha-Omega-Aminocarbonsäure abgeleitet ist, wobei das Lactam und/oder die Alpha-Omega-Aminocarbonsäure vorzugsweise zwischen 6 und 18 Kohlenstoffatomen umfasst,
- oder ein Muster U.V, welches aus der Kondensation von mindestens einem Diamin U mit mindestens einer zweiwertigen Carbonsäure V stammt, wobei

- das geradkettige oder verzweigte Diamin U unter einem aliphatischen Diamin, einem cycloaliphatischen Diamin, einem aromatischen Diamin und Mischungen davon ausgewählt ist und vorzugsweise zwischen 4 und 20 Kohlenstoffatomen umfasst,
- die geradkettige oder verzweigte zweiwertige Carbonsäure V unter einer zweiwertigen aliphatischen Säure, einer zweiwertigen cycloaliphatischen Säure, einer zweiwertigen aromatischen Säure und Mischungen davon ausgewählt ist und vorzugsweise zwischen 6 und 20 Kohlenstoffatomen umfasst.

10. Biomaterial nach Anspruch 8 oder 9, **dadurch gekennzeichnet, dass** die zweiwertige aromatische Carbonsäure Ar eine Phthalsäure ist, welche vorzugsweise unter Terephthalsäure, isophthalsäure, Orthophthalsäure und Mischungen davon ausgewählt ist.

11. Biomaterial nach einem der Ansprüche 9 bis 10, **dadurch gekennzeichnet, dass** die Anzahl von Kohlenstoffatomen von mindestens einem der Muster X und Y zwischen 6 und 12 Kohlenstoffatomen beträgt.

12. Biomaterial nach einem der Ansprüche 9 bis 11, **da-**

**durch gekennzeichnet, dass** mindestens eines von Muster Y und Diamin U unter der folgenden Gruppe ausgewählt ist: 1,6-Hexamethylendiamin, 1,9-Nonandiamin, 2-Methyl-1,8-octandiamin, 1,10-Decandiamin, 1,12-Dodecandiamin und Mischungen davon.

13. Biomaterial nach einem der Ansprüche 9 bis 12, **dadurch gekennzeichnet, dass** X unter Lactam 12, 11-Undecanaminosäure, 12-Dodecanaminosäure und Mischungen davon ausgewählt ist.

14. Biomaterial nach einem der Ansprüche 9 bis 13, **dadurch gekennzeichnet, dass** V unter der folgenden Gruppe ausgewählt ist: Adipinsäure, Suberinsäure, Azelainsäure, Sebacinsäure, zweiwertige 1,12-Dodecandisäure, Brassylsäure, zweiwertige 1,14-Tetradecandisäure, Terephthalsäure, Isophthalsäure, Naphthalendicarbonsäure und Mischungen davon.

15. Biomaterial nach einem der Ansprüche 9 bis 14, **dadurch gekennzeichnet, dass** die Molverhältnisse von X und Y (oder Ar) sind:

für Y = 1,
$0 \leq X \leq 0,7$.

16. Biomaterial nach Anspruch 15, **dadurch gekennzeichnet, dass** die Molverhältnisse von X und Y (oder Ar) vorzugsweise sind:

für Y = 1,
$0 \leq X \leq 0,5$.

17. Biomaterial nach einem der Ansprüche 9 bis 16, **dadurch gekennzeichnet, dass** die Diamine Y und U identisch sind.

18. Biomaterial nach einem der vorhergehenden Ansprüche, **dadurch gekennzeichnet, dass** die Masse der Verstärkung bis zu 70 % der Gesamtmasse des Biomaterials beträgt.

19. Biomaterial nach einem der vorhergehenden Ansprüche, **dadurch gekennzeichnet, dass** es darüber hinaus ein Tensid oder eine Mischung von Tensiden, ein amphiphiles Molekül oder eine Mischung von amphiphilen Molekülen oder einen beliebigen anderen Emulsionsvermittler oder Mischung von Emulsionsvermittlern umfasst.

20. Biomaterial nach einem der vorhergehenden Ansprüchen, **dadurch gekennzeichnet, dass** es hinzugefügtes Wasser umfasst, dessen Masse im Verhältnis zur Gesamtmasse des Biomaterials prozentual weniger als 5% beträgt.

**21.** Biomaterial nach einem der vorhergehenden Ansprüchen, **dadurch gekennzeichnet, dass** die halbaromatische Polyamidmatrix und die Verstärkung so ausgewählt sind, dass die dynamischen mechanischen Eigenschaften des Biomaterials einem Viskoelastizitätsniveau entspricht, welches bei Temperaturen und physiologischen Frequenzen in der Größenordnung von jenen liegt, die für ein verkalktes Gewebe kennzeichnend sind, definiert durch ein Speichermodul G' zwischen 100 MPa und 10 GPa (Grenzwerte eingeschlossen) im Torsionszustand und einen mechanischen Verlustfaktor, welcher durch tan $\delta$ wiedergegeben wird, höher als $10^{-3}$ im Torsionszustand.

**22.** Verwendung des Biomaterials nach einem der vorhergehenden Ansprüche für die Herstellung von Osteosynthesevorrichtungen oder Zahnprothesen.

**Figure 1**

**Figure 2**

**Figure 3**

**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- *conférence de Chester de la Société Européenne des Biomatériaux,* 1986